# EUROPÄISCHE PATENTANMELDUNG

(11) **EP 1 142 540 A2**
(43) Veröffentlichungstag der Anmeldung: **10.10.2001**
(21) Anmeldenummer: 01108357.3
(22) Anmeldetag: 03.04.2001
(51) Int. Cl.: A61C 5/06

(54) **Verfahren und Vorrichtung zum Abfüllen von dentalen Füllungsmaterialien**

(30) Priorität: 07.04.2000 DE 10017476
(71) Anmelder: Degussa Dental GmbH & Co. KG, 63457 Hanau (DE)
(72) Erfinder: Both, Adam, 63454 Hanau (DE); Kläres, Ulrich, 53347 Alfter (DE); Luckau, Ralf, 63796 Kahl (DE); Roth, Michael, 63814 Mainaschaff (DE); Bauer, Udo, 61130 Nidderau (DE)

(57) **Zusammenfassung**

Die Erfindung betrifft ein Verfahren sowie eine Vorrichtung zum Abfüllen von dentalen Füllungsmaterialien (2) in spezielle Einzelbehälter (3) für die zahnärztliche Anwendung. Ein Vorratsbehälter (1) wird dicht und druckfest mit einer konisch ausgeführten Düse (4) des Einzelbehälters (3) verbunden. Das dentale Füllungsmaterial (2) wird mittels Druck aus dem Vorratsbehälter (1) durch die Düse (4) in den Behälterkörper gepresst und schiebt dabei einen axial beweglichen Kolben (7) bis in eine durch einen regelbaren Anschlag (10) vorgegebene Endstellung vor sich her. Damit kann auch bei gleichzeitiger Abfüllung mehrerer Einzelbehälter (3) eine präzise Dosierung über eine lange Betriebsdauer gewährleistet werden. Ein im Bereich des Anschlags (10) angebrachter Sensor (11) registriert die erreichte Endstellung eines die Bewegung des Kolbens (7) übertragenden Anschlagsstößels (6).

## Beschreibung

Die Erfindung betrifft ein Verfahren zum Abfüllen von dentalen Füllungsmaterialien aus einem Vorratsbehälter in Einzelbehälter, welche einen Behälterkörper mit einer Düse an einem Ende sowie einen das andere Ende verschließenden, axial beweglichen Kolben aufweisen.

Für Anwendungen in einer Zahnarztpraxis werden dentalmedizinische Füllungsmaterialien, wie beispielsweise lichtaushärtende Füllungsmaterialien, in spezielle oft als Einwegbehälter verwendete Einzelbehälter abgefüllt. Solche Einzelbehälter beinhalten in der Regel eine für eine einmalige Anwendung ausreichende Menge des dentalen Füllungsmaterials, welches der Zahnarzt für die Restaurierung einer präparierten Zahnkavität benötigt.

Bei üblicherweise durchgeführten, bekannten Abfüllverfahren wird ein Einzelbehälter ohne zugehörigen Kolben so positioniert, daß eine aus dem Vorratsbehälter für dentales Füllungsmaterial herausragende Kanüle in den bei fehlendem Kolben an der entsprechenden Seite offenen Behälterkörper des zu befüllenden Einzelbehälters reicht. Durch Druck wird das dentale Füllungsmaterial aus dem Vorratsbehälter durch die Kanüle hindurch in den offenen Einzelbehälter gepreßt. Dabei kann die sich im Einzelbehälter befindende Austrittsöffnung der Kanüle entsprechend der zunehmenden Füllhöhe des dentalen Füllungsmaterials im Einzelbehälter nachgeführt werden. Nach Beendigung des Abfüllvorgangs wird der Einzelbehälter mit dem Kolben und einer auf die Düse gesteckten Kappe verschlossen.

Neben der jeweils abzufüllenden Menge des dentalen Füllungsmaterials hängt die dafür notwendige Abfüllzeit von Produkteigenschaften, beispielsweise der Viskosität des Füllungsmaterials, und Prozeßparametern, beispielsweise dem Abfülldruck, ab. Bei bekannten Abfüllverfahren wird das dentale Füllungsmaterial aus dem Vorratsbehälter durch dünne Austrittskanülen in die offenen Einzelbehälter gepreßt. Üblicherweise verwendete Austrittskanülen weisen einen geringen Durchmesser auf, so daß mit zunehmender Viskosität des dentalen Füllungsmaterials die Abfüllzeit unverhältnismäßig zunimmt bzw. der notwendige Abfülldruck so hoch gewählt werden muß, daß nachteilige Auswirkungen für das viskose Füllungsmaterial auftreten können. Aufgrund der hohen Beanspruchung der Austrittskanüle muß diese überwiegend oder ganz aus Metall bestehen. Der Kontakt abrasiver Füllungsmaterialien mit Metallflächen kann jedoch zu aufgenommenen Abriebpartikeln von der Austrittskanüle und dadurch verursachten Farbänderungen des abgefüllten Füllungsmaterials führen. Der Einsatz von Abfüllungsverfahren der eingangs genannten Gattung ist deshalb vor allem für hochviskose, pastenartige Füllungsmaterialien langwierig und unwirtschaftlich sowie mit bisher unvermeidbaren Nachteilen verbunden.

Aufgabe der Erfindung ist es daher, ein Verfahren zum Abfüllen dentaler Füllungsmaterialien der eingangs genannten Gattung so zu gestalten, daß auch hochviskose dentale Füllungsmaterialien schnell und ökonomisch abgefüllt werden können.

Diese Aufgabe wird erfindungsgemäß dadurch gelöst, daß das dentale Füllungsmaterial durch die Düse hindurch, den Kolben bis in eine vorgegebene Endstellung vor sich herschiebend, in den Behälterkörper eingepresst wird.

Zum Befüllen wird die Düse des Einzelbehälters dicht und druckfest abschließend an eine Austrittsöffnung des Vorratsbehälters angeschlossen. Der axial bewegliche Kolben befindet sich zu Beginn des Abfüllvorgangs im Einzelbehälter in der Nähe der Düse. Durch Druck wird dentales Füllungsmaterial durch die Düse hindurch in den Behälterkörper gepreßt und schiebt dabei den Kolben in Befüllungsrichtung vor sich her. Der Abfüllvorgang wird beendet, sobald der Kolben eine vorgegebene Endstellung erreicht. Die zu Beginn des Abfüllvorgangs im Einzelbehälter eingeschlossene Luft entweicht durch das Entlüftungsprofil einer Kolbendichtlippe, welche gleichzeitig einen ungewollten Austritt des dentalen Füllungsmaterials an der kolbenseitigen Öffnung des Einzelbehälters verhindert. Dadurch wird der Einzelbehälter vollständig und ohne Restluft mit dentalem Füllungsmaterial befüllt. Dies schließt vor allem die Düse mit ein, die mit dem bekannten Abfüllverfahren für hochviskose Füllungsmaterialien oftmals nicht oder nur unvollständig gefüllt wird. Als zusätzlicher Vorteil tritt während einer zahnärztlichen Behandlung sofort dentales Füllungsmaterial aus. Bisher musste zuerst durch mehrmaliges Betätigen des Applikators das Füllungsmaterial durch die zu Beginn leere Düse gepreßt werden, bevor es aus der Düse ausgetreten ist und für die Anwendung nutzbar wurde.

Vorzugsweise ist vorgesehen, daß die Endstellung des axial beweglichen Kolbens einstellbar ist. Über die einstellbare Endstellung des Kolbens wird das für dentales Füllungsmaterial zur Verfügung stehende Nutzvolumen des Einzelbehälters vorgegeben und damit die genaue Dosierung der Abfüllmenge mit hoher Präzision sichergestellt. Eine gewünschte Veränderung der abzufüllenden Dosis des dentalen Füllungsmaterials kann leicht durch eine veränderte Endstellung des Kolbens erreicht werden.

Gemäß einer Ausgestaltung des Erfindungsgedankens ist vorgesehen, daß aus dem Vorratsbehälter durch Austrittöffnungen mehrere Einzelbehälter simultan geregelt befüllt werden. Die in die Einzelbehälter abgefüllte Dosis des dentalen Füllungsmaterials ist durch die jeweilige Endstellung des Kolbens vorgegeben. Für eine Dosierung des dentalen Füllungsmaterials ist darüber hinaus keine zusätzliche Vorrichtung notwendig. Mit vergleichsweise geringem Aufwand können deshalb viele Einzelbehälter gleichzeitig präzise und reproduzierbar befüllt werden, was hinsichtlich der Abfülleistung und Wirtschaftlichkeit einen wesentlichen Vorteil bedeutet.

Gemäß einer bevorzugten Ausführungsform der Erfindung ist vorgesehen, daß nach einem Abfüllvorgang die tatsächlich in jeden Einzelbehälter abgefüllte Dosis des dentalen Füllungsmaterials durch Wägung kontrolliert und mittels eines Regelkreises vor einem darauffolgenden Abfüllvorgang der jeweils zugeordnete Kolbenhub entsprechend geregelt wird. Durch eine ständige Korrektur der Endstellung des Kolbens und damit der abzufüllenden Menge an dentalem Füllungsmaterial in Abhängigkeit von der tatsächlich gemessenen, im vorherigen Vorgang abgefüllten Dosis kann vollständig automatisiert auch über eine langen Betriebsdauer eine gleichbleibende Abfüllmenge des dentalen Füllungsmaterials gewährleistet werden.

Die Erfindung betrifft auch eine' Vorrichtung zur Durchführung des Verfahrens zum Abfüllen dentaler Füllungsmaterialien. Ausgehend von einer bekannten Vorrichtung mit einem einen Vorrat an dentalem Füllungsmaterial aufnehmenden Vorratsbehälter mit mindestens einer Austrittsöffnung ist die erfindungsgemäße Vorrichtung dadurch gekennzeichnet, daß die Austrittsöffnung als dicht und druckfest abschließende Kontaktstelle des Vorratsbehälters mit dem zu befüllenden Einzelbehälter ausgeführt ist und der Hub des axial im Behälterkörper beweglichen Kolbens durch einen Anschlag begrenzt ist.

Eine dichte und druckfeste Verbindung erlaubt einen solchen hohen Abfülldruck, mit welchem das dentale Füllungsmaterial aus dem Vorratsbehälter in den Einzelbehälter gepreßt wird, und reduziert damit die notwendige Abfüllzeit vor allem für hochviskose Füllungsmaterialien wesentlich.

Vorzugsweise ist vorgesehen, daß die Kontaktstelle als konische Bohrung im Vorratsbehälter ausgeführt ist, deren Form und Abmessungen der äußeren Form der Düse entsprechen. Aufgrund der konischen Ausführung sowohl der Austrittsöffnung im Vorratsbehälter als auch der Düse ist der Einzelbehälter einfach an der Kontaktstelle anschließbar. Allein durch Andrücken des Einzelbehälters an den Vorratsbehälter während des Abfüllvorgangs wird eine stabile und druckdichte Verbindung zwischen Vorratsbehälter und Einzelbehälter sichergestellt. Zusätzlich kann die Düse neben einer konischen Form auch eine etwa mittig angebrachte als ringförmige Einschnürung ausgestaltete Soll-Biegestelle aufweisen, die den Ausgleich eines möglichen Winkelversatzes des zum Abfüllen positionierten Behälters ermöglicht.

Gemäß einer bevorzugten Ausführungsform der Erfindung ist vorgesehen, daß sich im Bereich des Anschlags ein Sensor befindet, der bei der durch den Anschlag vorgegebenen Endstellung des Kolbens ein Signal auslöst. Damit wird automatisch das Beenden des Abfüllvorgangs eingeleitet. Auf dessen Signal hin kann das dentale Füllungsmaterial im Vorratsbehälter gegebenenfalls druckentlastet und der gefüllte Einzelbehälter aus der Kontaktstelle entnommen werden.

Gemäß einer Ausgestaltung des Erfindungsgedankens ist vorgesehen, daß ein in einer Führung gelagerter und mittels einer Feder in Richtung des Kolbens gedrückter, ebenso wie der Kolben axial beweglicher Anschlagsstößel bis in eine durch einen Anschlag vorgegebene Endstellung verschiebbar ist. Vor Beginn des Abfüllvorgangs wird der Anschlagsstößel in Richtung des Anschlags verschoben, so daß der Einzelbehälter zum Befüllen an der Kontaktstelle anschließbar ist. Danach wird der Anschlagsstößel entgegen der Befüllrichtung in den Einzelbehälter bis hin zu dem bereits eingelegten Kolben hineingeschoben. Durch Abfragen dieser Position kann die Anwesenheit des Kolbens geprüft werden. Die Federkraft verhindert, daß der Anschlagsstößel frühzeitig in die vorgegebene Endstellung verschoben wird und ungewollt ein den Abfüllvorgang beendendes Signal auslöst.

Vorzugsweise ist vorgesehen, daß der die Endstellung des Anschlagsstößels bzw. des Kolbens bestimmende Anschlag einstellbar ist. Auf diese Weise wird mit nur geringem konstruktivem Mehraufwand eine genaue und reproduzierbare Dosierung schon während des Abfüllvorgangs erreicht. Mit der Endstellung des Kolbens kann mit geringem Zeitaufwand die gewünschte Dosis des jeweils abzufüllenden dentalen Füllungsmaterials verändert werden, so daß nur kurze Maschinenstillstandszeiten für die Umrüstung von einem Abfüllgewicht auf ein anderes notwendig sind.

Gemäß einer bevorzugten Ausführung des Erfindungsgedankens ist vorgesehen, daß der die Endstellung des Anschlagsstößels bzw. des Kolbens bestimmende Anschlag mittels eines Regelkreises automatisch regelbar ist. Nach erfolgter Befüllung eines Einzelbehälters kann die tatsächlich abgefüllte Dosis des dentalen Füllungsmaterials gemessen werden und in Abhängigkeit von dem Meßwert die den Kolbenhub begrenzende Endstellung korrigiert werden. Damit ist auch über einen langen Betriebszeitraum eine kontrollierte und präzise Befüllung vieler Einzelbehälter durchführbar.

Nachfolgend werden Ausführungsbeispiele der Erfindung näher erläutert, die in der Zeichnung dargestellt sind. Es zeigt:
Fig. 1 eine Vorrichtung zum Abfüllen von dentalem Füllungsmaterial in einen Einzelbehälter,
Fig. 2 einen Schnitt durch den Einzelbehälter vor der Befüllung,
Fig. 3 einen Schnitt durch den Einzelbehälter während des Abfüllvorgangs und
Fig. 4 eine Vorrichtung zum gleichzeitigen Abfüllen von dentalem Füllungsmaterial in mehrere Einzelbehälter, wobei die Endstellung der Kolben einstellbar ist.

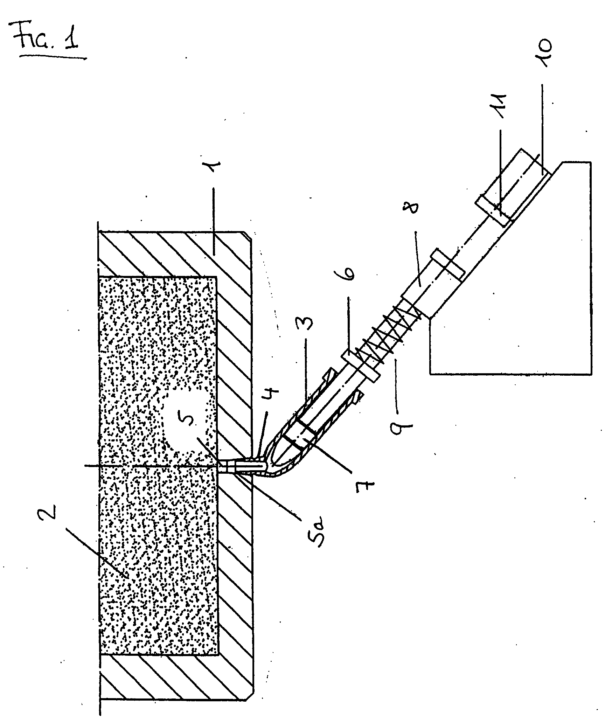

Die in Fig. 1 dargestellte Vorrichtung weist einen Vorratsbehälter 1 auf, in welchem sich ein Vorrat von abzufüllendem dentalem Füllungsmaterial 2 befindet. Ein zu befüllender Einzelbehälter 3 ist so positioniert, daß dessen Düse 4 dicht und druckfest an eine als konische Bohrung 5a ausgeführte Austrittsöffnung 5 des Vorratsbehälters 1 anschließt. Ein zylindrischer Anschlagsstößel 6, dessen Durchmesser angenähert dem des Kolbens 7 entspricht, ist in einer Führung 8 so gelagert, daß er ebenso wie der Kolben 7 in axialer Richtung im Einzelbehälter 3 verschiebbar ist. Eine Feder 9 drückt den Anschlagsstößel 6 in Richtung zur Düse 4 bis zu dem dort befindlichen Kolben 7. Wird mittels Druck das dentale Füllungsmaterial 2 aus dem Vorratsbehälter 1 durch die Austrittsöffnung 5 und die Düse 4 in den Einzelbehälter 3 gepreßt, so wird dadurch dem zunehmenden Volumen des dentalen Füllungsmaterials 2 im Einzelbehälter 3 entsprechend der Kolben 7 und damit der Anschlagsstößel 6 gegen die Wirkung der Federkraft verschoben. Zunächst entweicht die Luft, dann wird das Volumen allmählich gefüllt, wobei der Kolben 7 nach hinten bewegt wird. Wenn der Kolben 7 nicht weitergedrückt werden kann, füllt das nachfließende Füllungsmaterial den Restraum um die Kolbenspitze aus. Ein Anschlag 10 definiert in dieser Richtung die Endstellung des Anschlagsstößels 6 bzw. des Kolbens 7. In der vorgegebenen Endstellung befindet sich der Kolben 7 noch innerhalb des Einzelbehälters 3 und schließt diesen dicht ab. Mit einem Sensor 11, der sich im Bereich des Anschlags 10 befindet, kann die erreichte Endstellung des Anschlagsstößels 6 und damit die Beendigung des Befüllungsvorgangs eingeleitet werden.

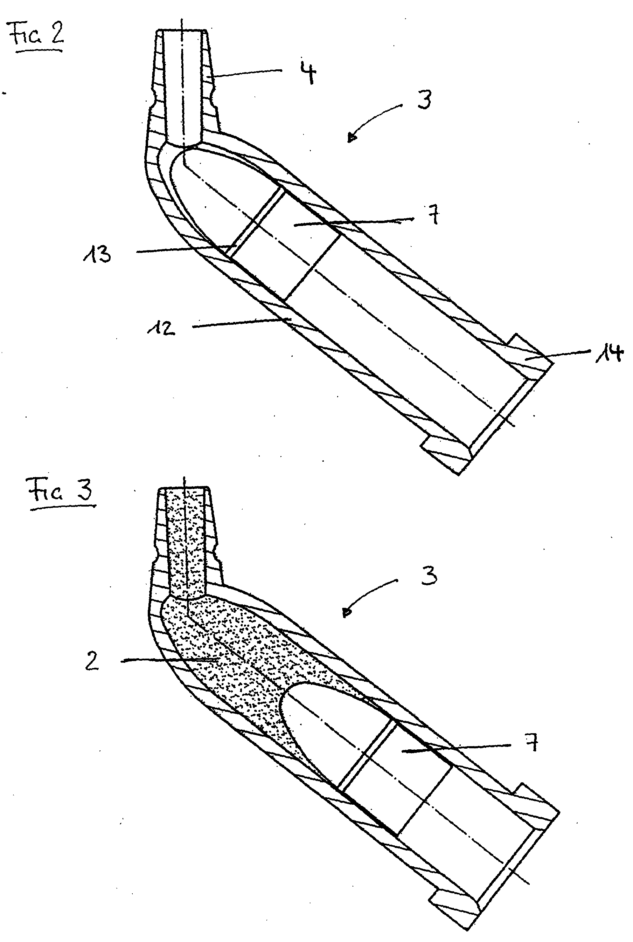

In Fig. 2 ist der Einzelbehälter 3 vor Beginn eines Abfüllvorgangs dargestellt. Das eine Ende des hohlzylindrischen Behälterkörpers 12 ist als seitlich abgewinkelte Düse 4 ausgeführt. Im Behälterkörper 12 befindet sich der axial bewegliche Kolben 7 mit einer radial vorspringenden, mit einem Entlüftungsprofil versehenen Kolbendichtlippe 13. Das der Düse 4 entgegengesetzte Ende des hohlzylindrischen Behälterkörpers 12 weist einen radial vorspringenden Behälterrand 14 auf, der einer besseren Druckfestigkeit des Behälterkörpers 12 dient und den Einzelbehälter 3 während des Abfüllvorgangs und im Applikator positioniert.

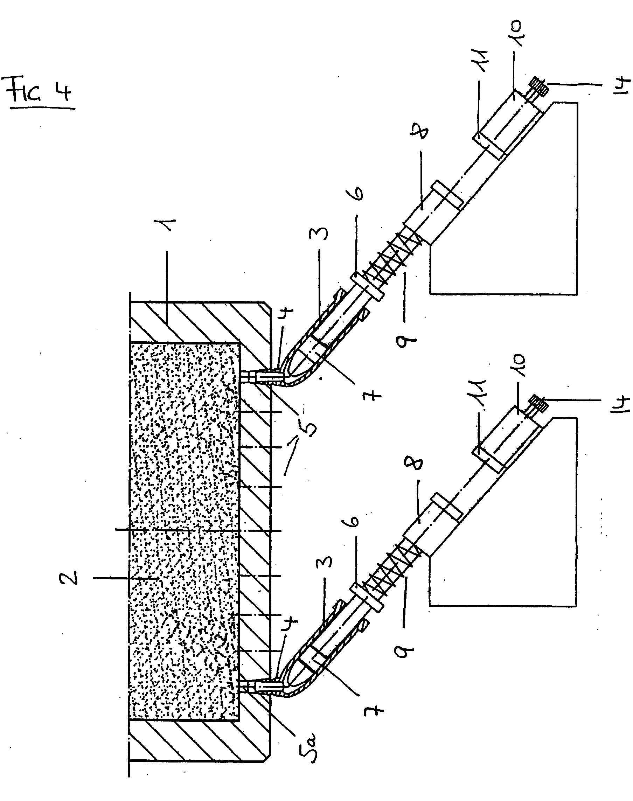

Fig. 3 zeigt den Einzelbehälter 3 während des Abfüllvorgangs. Das unter Druck stehende dentale Füllungsmaterial 2 dringt durch die Düse 4 in den Behälterkörper 12 ein und schiebt dabei den dicht abschließenden Kolben 7 in Befüllungsrichtung vor sich her.

Die beispielhaft für das gleichzeitige Abfüllen mehrerer Einzelbehälter 3 in Fig. 4 gezeigte Ausführung weist Austrittsöffnungen 5 des Vorratsbehälters 1 auf, wobei nur für zwei Austrittsöffnungen 5 Einzelheiten dargestellt sind. An den Austrittsöffnungen 5 ist jeweils ein Einzelbehälter 3 dicht abschließend angeordnet. In jedem Einzelbehälter 3 wird der zylindrische Anschlagsstößel 6 mittels Federkraft in Richtung der Austrittsöffnung 5 gedrückt. In entgegengesetzter Richtung ist der Hub des Anschlagsstößels 6 durch den Anschlag 10 begrenzt. Diese Endstellung kann für jeden Anschlagsstößel 6 individuell, beispielsweise mittels einer Schraubverstellung 14 geregelt werden. Die befüllten Einzelbehälter 3 können nach einem Abfüllvorgang jeweils einzeln gewogen werden. In Abhängigkeit der tatsächlich abgefüllten Menge an dentalem Füllungsmaterial ist damit für jede Austrittsöffnung 5 eine solch individuelle Regelung der Endstellung der zugeordneten Anschlagsstößel 6 möglich.

## Patentansprüche

1. Verfahren zum Abfüllen von dentalen Füllungsmaterialien aus einem Vorratsbehälter in Einzelbehälter, welche einen Behälterkörper mit einer Düse an einem Ende sowie einen das andere Ende verschließenden, axial beweglichen Kolben aufweisen,
**dadurch gekennzeichnet,**
**daß** das dentale Füllungsmaterial (2) durch die Düse (4) hindurch, den Kolben (7) bis in eine vorgegebene Endstellung vor sich herschiebend, in den Behälterkörper (12) eingepresst wird.

2. Verfahren nach Anspruch 1,
**dadurch gekennzeichnet,**
**daß** die Endstellung des axial beweglichen Kolbens (7) einstellbar ist.

3. Verfahren nach Anspruch 1 und 2,
**dadurch gekennzeichnet,**
**daß** aus dem Vorratsbehälter (1) durch Austrittsöffnungen (5) mehrere Einzelbehälter (3) simultan geregelt befüllt werden.

4. Verfahren nach Anspruch 3,
**dadurch gekennzeichnet,**
**daß** nach einem Abfüllvorgang die tatsächlich in jeden Einzelbehälter (3) abgefüllte Dosis des dentalen Füllungsmaterials (2) durch Wägung kontrolliert und mittels eines Regelkreises vor einem darauffolgenden Abfüllvorgang der jeweils zugeordnete Kolbenhub entsprechend geregelt wird.

5. Vorrichtung zur Durchführung des Verfahrens zum Abfüllen von dentalem Füllungsmaterial nach Anspruch 1 mit einem einen Vorrat an dentalem Füllungsmaterial aufnehmenden Vorratsbehälter mit mindestens einer Austrittsöffnung,
**dadurch gekennzeichnet,**
**daß** die Austrittsöffnung (5) als dicht und druckfest abschließende Kontaktstelle des Vorratsbehälters (1) mit dem zu befüllenden Einzelbehälter (3) ausgeführt ist und der Hub des axial im Behälterkörper (12) beweglichen Kolbens (7) durch einen Anschlag (10) begrenzt ist.

6. Vorrichtung nach Anspruch 5,
**dadurch gekennzeichnet,**
**daß** die Kontaktstelle als konische Bohrung (5a) im Vorratsbehälter (1) ausgeführt ist, deren Form und Abmessungen der äußeren Form der Düse (4) entsprechen.

7. Vorrichtung nach Anspruch 5,
**dadurch gekennzeichnet,**
**daß** sich im Bereich des Anschlags (10) ein Sensor (11) befindet, der bei der durch den Anschlag (10) vorgegebenen Endstellung des Kolbens (7) ein Signal auslöst.

8. Vorrichtung nach Anspruch 5,
**dadurch gekennzeichnet,**
**daß** ein in einer Führung (8) gelagerter und mittels einer Feder (9) in Richtung des Kolbens (7) gedrückter ebenso wie der Kolben (7) axial beweglicher Anschlagsstößel (6) bis in eine durch einen Anschlag (10) vorgegebene Endstellung verschiebbar ist.

9. Vorrichtung nach Anspruch 8,
**dadurch gekennzeichnet,**
**daß** daß der die Endstellung des Anschlagsstößel (6) bzw. des Kolbens (7) bestimmende Anschlag (10) einstellbar ist.

10. Vorrichtung nach Anspruch 9,
**dadurch gekennzeichnet,**
**daß** der die Endstellung des Anschlagsstößel (6) bzw. des Kolbens (7) bestimmende Anschlag (10) mittels eines Regelkreises automatisch regelbar ist.
